# EUROPÄISCHE PATENTSCHRIFT

(11) **EP 3 339 134 B1**
(45) Veröffentlichungstag und Bekanntmachung des Hinweises auf die Patenterteilung: **25.03.2020**
(21) Anmeldenummer: 17205055.1
(22) Anmeldetag: 04.12.2017
(51) Int. Cl.: B62B 5/00

(54) **HOHLPROFILEINSTECKSTOPFEN**
INSERTION PLUG FOR HOLLOW PROFILE
BOUCHON D'INSERTION POUR PROFILE CREUX

(30) Priorität: 21.12.2016 DE 102016125153
(43) Veröffentlichungstag der Anmeldung: 27.06.2018
(73) Patentinhaber: Wanzl GmbH & Co. KGaA, 89340 Leipheim (DE)
(72) Erfinder: STUPKA, Karel, CZ 78391 Unicov (CZ); SAFRANEK, Stefan, CZ 79823 Klenovice na Hané (CZ)

(56) Entgegenhaltungen:
- EP-A1- 2 868 610
- DE-A1-102007 055 868
- DE-U1- 20 317 367
- JP-A- 2012 116 555
- NL-C- 1 038 737

## Beschreibung

Die vorliegende Erfindung betrifft ein Hohlprofileinsteckstopfen mit einem Grundkörper zum Einsatz in ein offenes Ende eines Hohlprofils und mit einer Abdeckseite zur Abdeckung des offenen Endes des Hohlprofils.

Derartige Hohlprofileinsteckstopfen sind beispielsweise Im Zusammenhang mit Fahrgestellen oder fahrbaren Gestellen bekannt. Dabei werden die Fahrgestelle üblicherweise aus mehreren Hohlprofilen ausgebildet, die beispielsweise miteinander verschraubt oder verschweißt sind. Die offenen Enden der Hohlprofile werden sodann mit Hohlprofileinsteckstopfen verschlossen. Die Profllabschlusskappen oder Hohlprofileinsteckstopfen werden auch als Prallschutz für ineinander stapelbare bzw. ineinander schiebbare Wagen oder Fahrgestelle verwendet.

Beispielsweise sind entsprechende Hohlprofileinsteckstopfen bereits aus den europäischen Gemeinschaftsgeschmacksmustern EU-GGM 002325613-0003, 003421403-0001, 003421403-0002, 003424043-0001 und 003424043-0002 bekannt.

Weiterhin ist aus der NL 1 038 737 C ein Hohlprofileinsteckstopfen mit einem Grundkörper bekannt. Dieser dient zum Einsatz in ein offenes Ende eines Hohlprofils und mit einer Abdeckseite zur Abdeckung des offenen Endes des Hohlprofils, wobei die Abdeckseite an den Grundkörper angeformt ist und einen umlaufenden Überstand hat. Angrenzend an den Überstand im Übergang zur angrenzenden Außenfläche des Grundkörpers ist eine Einsenkung vorgesehen.

Auch die deutsche Patentanmeldung 10 2007 055 868 A1 offenbart einen solchen Hohlprofileinsteckstopfen, der mit einer Abdeckseite, einem umlaufenden Überstand und einer Einsenkung versehen Ist.

Diese Hohlprofileinsteckstopfen sind jedoch aus einem vergleichsweise spröden und festen Material ausgebildet und weisen daher bei der Montage sowie in Bezug auf die Haltbarkeit Nachteile auf.

Es ist daher die Aufgabe der vorliegenden Erfindung, einen Hohlprofileinsteckstopfen der eingangs genannten Art in vorteilhafter Weise weiterzubilden, insbesondere dahingehend, dass dieser leichter montierbar ist, eine längere Haltbarkeit aufweist und darüber hinaus Fertigungsungenauigkeiten ausgleichen kann.

Diese Aufgabe wird erfindungsgemäß gelöst durch einen Hohlprofileinsteckstopfen mit den Merkmalen des Anspruchs 1. Danach Ist vorgesehen, dass ein Hohlprofileinsteckstopfen mit einem Grundkörper zum Einsatz In ein offenes Ende eines Hohlprofils und mit einer Abdeckseite zur Abdeckung des offenen Endes des Hohlprofils versehen ist, wobei die Abdeckseite an den Grundkörper angeformt ist und einen umlaufenden Überstand hat, wobei angrenzend an den Überstand im Übergang zur angrenzenden Außenfläche des Grundkörpers eine Einsenkung vorgesehen ist, wobei im Grundkörper eine Stangenaufnahme ausgebildet ist, und wobei die Stangenaufnahme rohrförmig in den Grundkörper eingelassen ist.

Die Erfindung basiert auf dem Grundgedanken, dass zwischen dem Übergang von Abdeckseite zum Grundkörper keine im Wesentlichen rechtwinklige Kante vorgesehen ist, sondern dass an dieser Stelle eine Einsenkung vorgesehen ist. Durch diese Einsenkungen wird es vermieden, dass zwischen dem umlaufenden Überstand und im Übergang zur angrenzenden Außenfläche des Grundkörpers fertigungsbedingt Grate oder dergleichen entstehen können und diese entsprechend entfernt werden müssen. Durch die Einsenkung wird bereits durch die Formgebung darüber hinaus eine gewisse Flexibilität dem Hohlprofileinsteckstopfen zugewiesen, die sich Insbesondere in einer gewissen Beweglichkeit zwischen der Abdeckseite und dem Grundkörper äußert. Die Abdeckseite selbst dient der Abdeckung des offenen Endes des Hohlprofils. Die Abdeckseite ist dabei bei eingestecktem Zustand des Hohlprofileinsteckstopfens die nach außen hin ersichtliche Seite des Hohlprofileinsteckstopfens im Hohlprofil. Der Überstand selbst steht über die Außenflächen des Grundkörpers hinaus, was insbesondere in der Seitenansicht gut ersichtlich ist. Dieser Überstand dient dazu, sich auf die Kanten des Hohlprofils aufzulegen und diese entsprechend zu überdecken bzw. abzudecken.

Darüber hinaus kann vorgesehen sein, dass die Einsenkung umlaufend ausgebildet ist. Durch eine umlaufende Einsenkung wird erreicht, dass die formbedingte Flexibilität gleichmäßig um die Abdeckseite herum gegeben ist und nach allen Seiten eine entsprechende Flexibilität gegebenenfalls bereitgestellt werden kann.

Des Weiteren ist möglich, dass der Hohlprofileinsteckstopfen einstückig ausgebildet ist. Hierdurch wird eine einfache Fertigung erreicht.

Insbesondere ist weiter denkbar, dass der Hohlprofileinsteckstopfen als Spritzgussteil ausgebildet ist. Hierdurch kann eine einfache und kostengünstige, gleichzeitig aber hoch qualitative Fertigung erreicht werden.

Bei einer Fertigung als Spritzgussteil lassen sich die Einsenkungen insbesondere auch nutzen, um Fertigungsgrate zu verhindern. Insbesondere im Zusammenhang mit der Verwendung eines flexiblen Werkstoffs ist auch eine Entformung aus einer Spritzgussform unproblematisch.

Der Hohlprofileinsteckstopfen kann zumindest teilweise aus Kunststoff ausgebildet sein. Dabei kann es sich insbesondere um ein Kunststoffspritzgussteil handeln.

Insbesondere ist dabei denkbar, dass der Kunststoff ein elastischer und/oder flexibler Kunststoff ist. Dabei sind insbesondere Kunststoffe auf Silikonbasis, Polytetrafluorethylen (PTFE) und Polyvinylidenfluorid (PVDF) Kunststoffe denkbar. Denkbar ist auch, Polyvinylchlorid (PVC) zu benutzen. Denkbar ist auch, Polyamide zu verwenden.

Auf wenigstens einer Außenfläche des Grundkörpers kann eine Verrastungsnoppe angeordnet sein. Mittels der Verrastungsnoppe kann erreicht werden, dass der Hohlprofileinsteckstopfen beim Einstecken in das Hohlprofil verrastet werden kann. Denkbar ist, dass auch mehrere Verrastungsnoppen auf den Außenflächen des Grundkörpers vorgesehen sind.

Darüber hinaus ist denkbar, dass im Grundkörper eine Stangenaufnahme ausgebildet ist. Die Stangenaufnahmen kann beispielsweise benutzt werden, um Aufbauten, Stangen für Schiebegriffe oder dergleichen im Hohlprofil zu befestigen. Durch eine derartige Konstruktion wird es beispielsweise möglich, eine Stange durch entsprechende Öffnungen im Hohlprofil auch durch den Hohlprofileinsteckstopfen hindurch bzw. teilweise durch den Hohlprofileinsteckstopfen hindurchzustecken, und somit diese drei Elemente miteinander bzw. aneinander zu befestigen.

Die Stangenaufnahme kann dabei klammerartig ausgebildet sein.

Dabei kann die Klammer beispielsweise durch zwei Klammerfinger, die flexibel ausgebildet sind, bereitgestellt werden. Denkbar ist in diesem Zusammenhang beispielsweise, dass ein Klammerfinger sich beispielsweise an die Innenwand des Hohlprofils anlegen kann, während der zweite Klammerfinger frei beweglich im Hohlprofil angeordnet ist.

Denkbar ist aber auch, dass die Stangenaufnahme rohrförmig in den Grundkörper eingelassen ist. In diesem Fall wird eine Stange durch den Grundkörper selbst in die Stangenaufnahme und durch die Stangenaufnahme hindurch hineingesteckt.

Weitere Einzelheiten und Vorteile der Erfindung sollen nun anhand von in den Zeichnungen dargestellten Ausführungsbeispielen näher erläutert werden.

Es zeigen:
- Fig. 1: eine Draufsicht auf ein erstes Ausführungsbeispiel eines erfindungsgemäßen Hohlprofileinsteckstopfens;
- Fig. 2: eine perspektivische Ansicht auf den Hohlprofileinsteckstopfen gemäß Fig. 1;
- Fig. 3: eine Draufsicht auf ein weiteres Ausführungsbeispiel eines erfindungsgemäßen Hohlprofileinsteckstopfens;
- Fig. 4: eine perspektivische Ansicht des Hohlprofileinsteckstopfens gemäß Fig. 3;
- Fig. 5: ein Fahrgestell bestehend aus Hohlprofilen mit den Hohlprofileinsteckstopfen gem. Fig. 1-4;
- Fig. 6: eine Draufsicht auf ein weiteres Ausführungsbeispiel eines erfindungsgemäßen Hohlprofileinsteckstopfens;
- Fig. 7: eine perspektivische Ansicht auf den Hohlprofileinsteckstopfen gemäß Fig. 6;
- Fig. 8: eine Draufsicht auf ein weiteres Ausführungsbeispiel eines erfindungsgemäßen Hohlprofileinsteckstopfens; und
- Fig. 9: eine perspektivische Ansicht des Hohlprofileinsteckstopfens gemäß Fig. 8.

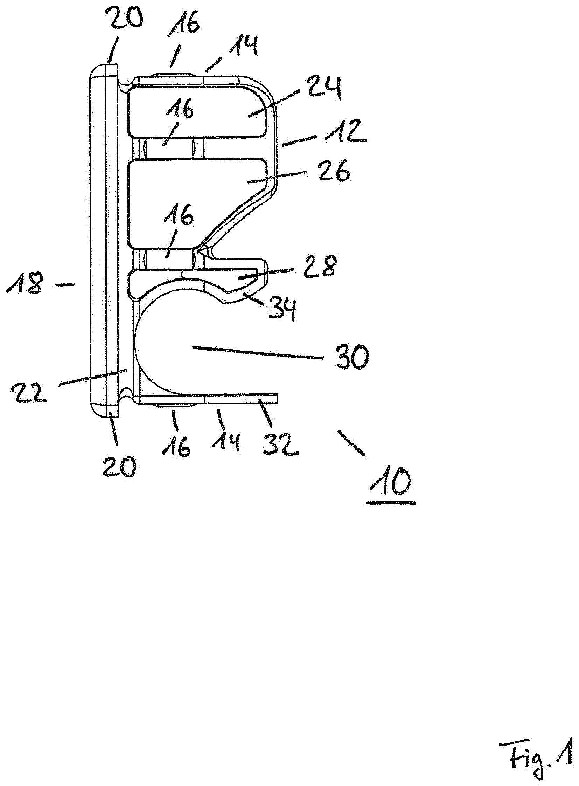

Fig. 1 zeigt in perspektivischer Ansicht einen Hohlprofileinsteckstopfen 10.

Der Hohlprofileinsteckstopfen 10 ist dabei einstückig ausgebildet.

Der Hohlprofileinsteckstopfen 10 besteht dabei aus Kunststoff und ist als Kunststoffspritzgussteil ausgebildet.

Bei dem Kunststoff handelt es sich um einen elastischen und flexiblen Kunststoff, hier Polyamid.

Denkbar ist auch, andere Kunststoffe, wie Polytetrafluorethylen (PTFE), Polyvinylidenfluorid (PVDF), Polyvinylchlorid (PVC) oder andere geeignete Kunststoffe, beispielsweise auf Silikonbasis, zu verwenden.

Der Hohlprofileinsteckstopfen 10 weist einen Grundkörper 12 mit Außenflächen 14 auf. In den Außenflächen 14 ist an mehreren Stellen eine Verrastungsnoppe 16 ausgebildet. Mittels dieser Verrastungsnoppen 16 wird eine Verrastung im Inneren des Hohlprofils erreicht.

Der Hohlprofileinsteckstopfen 10 weist weiter eine Abdeckseite 18 auf, wobei die Abdeckseite an den Grundkörper 12 einstückig angeformt ist.

Darüber hinaus weist die Abdeckseite 18 einen umlaufenden Überstand 20 auf, der sich deutlich vom Grundkörper 12 abhebt und über diesen übersteht.

Angrenzend an den Überstand 20 ist im Übergang zur angrenzenden Außenfläche 14 des Grundkörpers 12 eine Einsenkung 22 vorgesehen.

Die Einsenkung 22 ist im gezeigten Ausführungsbeispiel umlaufend ausgebildet.

Wie dies weiter in der Draufsicht ersichtlich ist, sind im Grundkörper 12 mehrere Ausnehmungen 24, 26 und 28 vorgesehen.

Durch diese Ausnehmungen 24, 26 und 28 wird erreicht, dass der Grundkörper im Wesentlichen in seinem Inneren durch Wandungen bzw. Stege ausgebildet ist, die gegeneinander aufgrund der Formgebung flexibel sind. Der Grundkörper 12 wird hierdurch insgesamt flexibel ausgebildet.

Der Grundkörper 12 weist weiter, ebenfalls durch eine Ausnehmung ausgebildet, eine Stangenaufnahme 30 auf.

Die Stangenaufnahme 30 wird dabei durch elastische Finger 32 und 34 ausgebildet, die dazu dienen, eine in den Grundkörper 12 und die Stangenaufnahme 30 eingesteckte Stange zu umgreifen. Aufgrund der Ausnehmung 28 ist der Finger 34 in der Lage, sich federnd an eine in die Stangenaufnahme 30 eingesetzte Stange anzulegen.

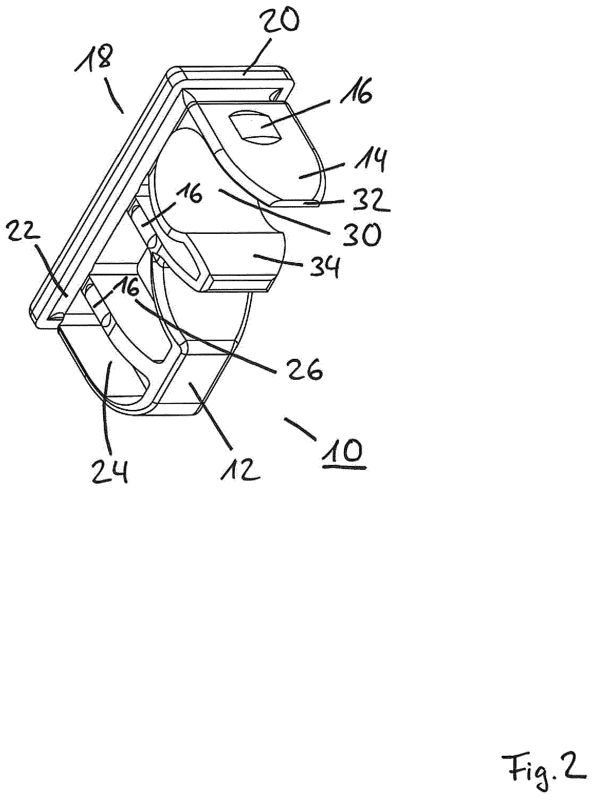

Fig. 2 zeigt in perspektivischer Ansicht den in Fig. 1 gezeigten Hohlprofileinsteckstopfen 10.

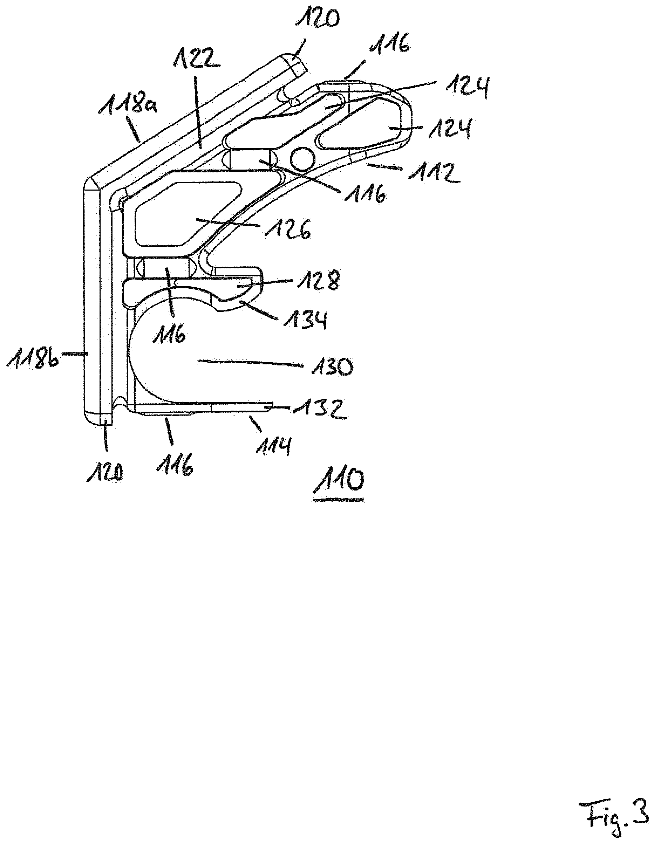

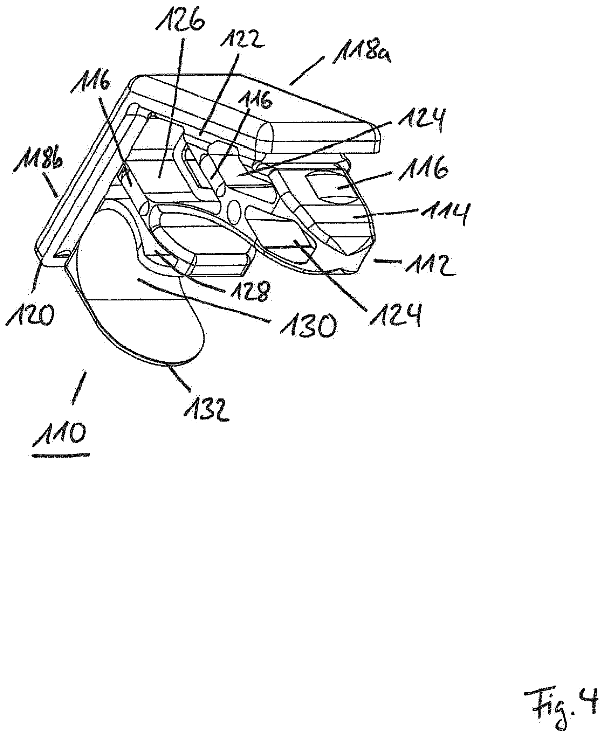

Fig. 3 und Fig. 4 zeigen ein weiteres Ausführungsbeispiel eines Hohlprofileinsteckstopfens 110.

Der Hohlprofileinsteckstopfen 110 weist dabei sämtliche strukturellen und funktionalen Merkmale wie auch der Hohlprofileinsteckstopfen 10 gemäß Fig. 1 und Fig. 2 auf.

Die entsprechenden Merkmale sind hier nur mit einem um den Wert 100 erhöhtes Bezugszeichen in den Figuren 3 und 4 kenntlich gemacht.

Ein Unterschied besteht insbesondere darin, dass die Abdeckseite 118 hier zweigeteilt ist in die Abdeckseiten 118a und 118b.

Die Abdeckseiten 118a und 118b sind dabei winklig zueinander angestellt und bilden einen stumpfen Winkel aus.

Der Winkel kann dabei beispielsweise im Bereich von ca. 130° bis 150° gewählt sein. Auch die umlaufende Einsenkung 122 ist dabei entsprechend der Anwinklung der Abdeckseiten 118a und 118b angewinkelt, jedoch ebenfalls vollständig umlaufend ausgebildet.

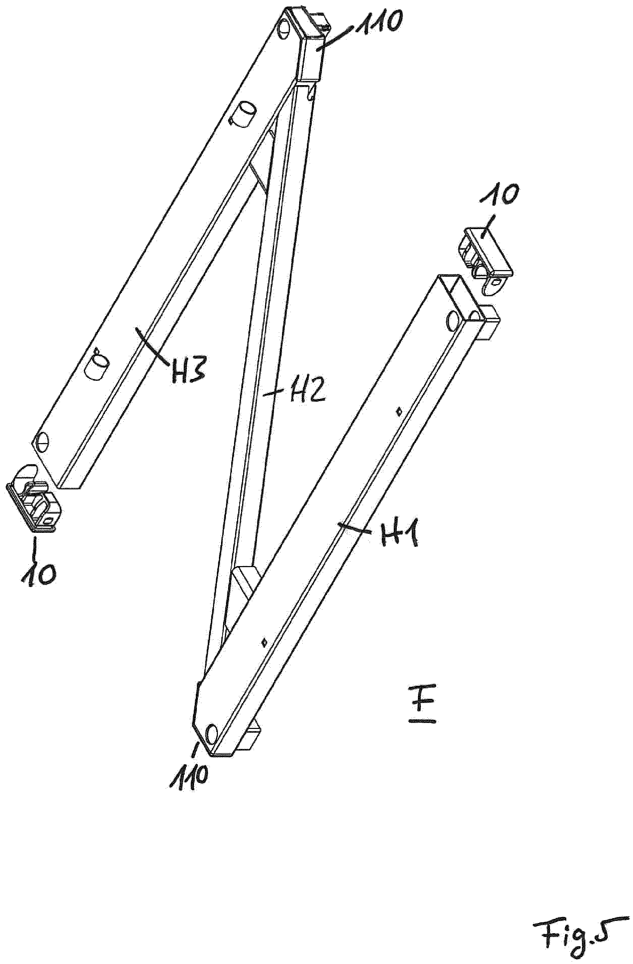

Fig. 5 zeigt in perspektivischer Ansicht ein Fahrgestell F mit Hohlprofilen H1, H2 und H3, die insgesamt ein N-förmiges Fahrgestell F ausbilden.

Die endseitigen Öffnungen der Hohlprofile H1 und H3 werden dabei mit den Hohlprofileinsteckstopfen 10 bzw. 110 verschlossen.

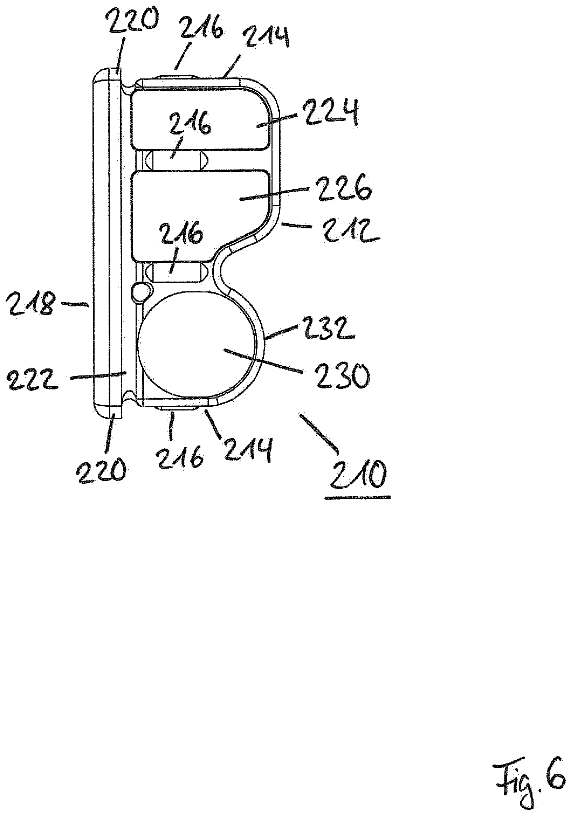

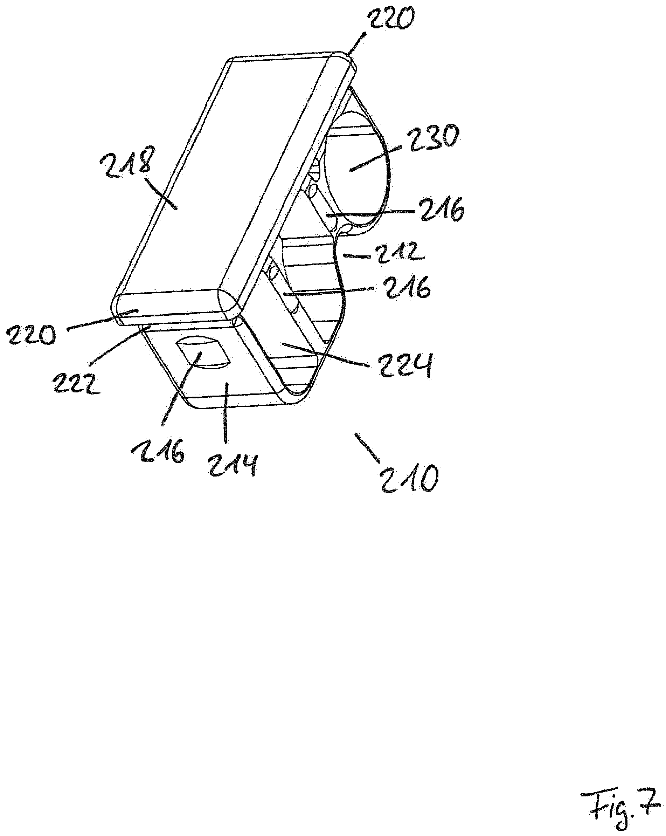

In den Fig. 6 und Fig. 7 ist weiter ein leicht abgewandeltes Ausführungsbeispiel eines Hohlprofileinsteckstopfens 210 gezeigt.

Der Hohlprofileinsteckstopfen 210 weist dabei ebenfalls sämtliche strukturellen und funktionalen Merkmale wie der Hohlprofileinsteckstopfen gemäß dem Ausführungsbeispiel nach Fig. 1 und Fig. 2 auf.

Entsprechend sind identische Merkmale mit um den Wert 200 erhöhte Bezugszeichen in den Fig. 6 und 7 gezeigt.

Die Stangenaufnahme 230 ist hier jedoch rohrförmig in den Grundkörper 212 eingelassen.

Der Wandabschnitt 232 bildet eine Ringwandung aus, so dass sich insgesamt die rohrförmige Stangenaufnahme 230 ausbildet.

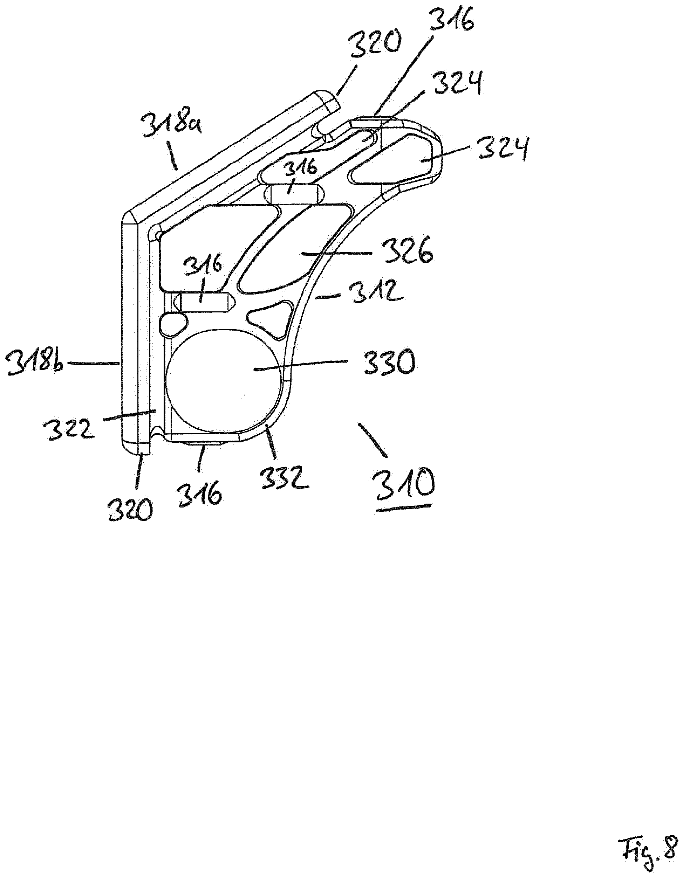

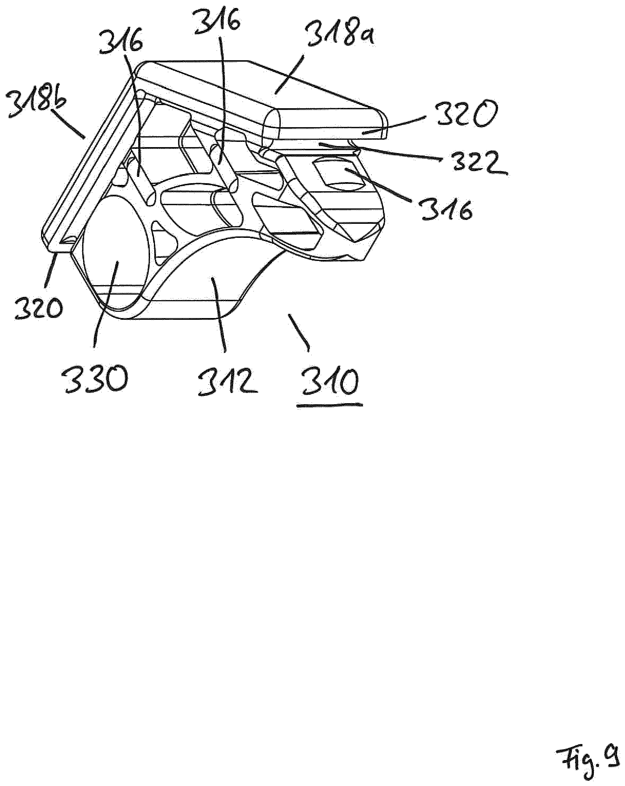

In den Fig. 8 und Fig. 9 ist ein weiteres Ausführungsbeispiel eines Hohlprofileinsteckstopfens 310 gezeigt. Der Hohlprofileinsteckstopfen 310 entspricht dabei im Wesentlichen dem in Fig. 3 und Fig. 4 gezeigten Ausführungsbeispiels des Hohlprofileinsteckstopfens 110.

Entsprechend sind auch hier die Bezugszeichen um den Wert 200 im Vergleich zu dem Ausführungsbeispiel gemäß Fig. 3 und Fig. 4 erhöht.

Analog dem in Fig. 6 und Fig. 7 gezeigten Ausführungsbeispiel ist aber auch hier eine rohrförmige Stangenaufnahme 330 vorgesehen.

Die Stangenaufnahme 330 ist hier ebenfalls rohrförmig in den Grundkörper 212 eingelassen.

Der Wandabschnitt 332 bildet auch hier eine Ringwandung aus, so dass sich insgesamt die rohrförmige Stangenaufnahme 330 ausbildet.

### Bezugszeichen

- 10: Hohlprofileinsteckstopfen
- 12: Grundkörper
- 14: Außenfläche
- 16: Verrastungsnoppen
- 18: Abdeckseite
- 20: Überstand
- 22: Einsenkung
- 24: Ausnehmung
- 26: Ausnehmung
- 28: Ausnehmung
- 30: Stangenaufnahme
- 32: Finger
- 34: Finger

- 110: Hohlprofileinsteckstopfen
- 112: Grundkörper
- 114: Außenfläche
- 116: Verrastungsnoppen
- 118a: Abdeckseite
- 118b: Abdeckseite
- 120: Überstand
- 122: Einsenkung
- 124: Ausnehmung
- 126: Ausnehmung
- 128: Ausnehmung
- 130: Stangenaufnahme
- 132: Finger
- 134: Finger

- 210: Hohlprofileinsteckstopfen
- 212: Grundkörper
- 214: Außenfläche
- 216: Verrastungsnoppen
- 218: Abdeckseite
- 220: Überstand
- 222: Einsenkung
- 224: Ausnehmung
- 226: Ausnehmung
- 228: Ausnehmung
- 230: Stangenaufnahme
- 232: Wandabschnitt

- 310: Hohlprofileinsteckstopfen
- 312: Grundkörper
- 314: Außenfläche
- 316: Verrastungsnoppen
- 318a: Abdeckseite
- 318b: Abdeckseite
- 320: Überstand
- 322: Einsenkung
- 324: Ausnehmung
- 326: Ausnehmung
- 328: Ausnehmung
- 330: Stangenaufnahme
- 332: Wandabschnitt

- F: Fahrgestell
- H1: Hohlprofil
- H2: Hohlprofil
- H3: Hohlprofil

## Patentansprüche

1. Hohlprofileinsteckstopfen (10; 110; 210; 310) mit einem Grundkörper (12; 112; 212; 312) zum Einsatz in ein offenes Ende eines Hohlprofils und mit einer Abdeckseite (18; 118a; 118b; 218; 318a; 318b) zur Abdeckung des offenen Endes des Hohlprofils, wobei die Abdeckseite (18; 118a; 118b; 218; 318a; 318b) an den Grundkörper (12; 112; 212; 312) angeformt Ist und einen umlaufenden Überstand (20; 120; 220; 320) hat, wobei angrenzend an den Überstand (20; 120; 220; 320) Im Übergang zur angrenzenden Außenfläche (14; 114; 214; 314) des Grundkörpers (12; 112; 212; 312) eine Einsenkung (22; 122; 222; 322) vorgesehen ist,
wobei im Grundkörper (12; 112; 212; 312) eine Stangenaufnahme (30; 130; 230; 330) ausgebildet ist,
und wobei die Stangenaufnahme (230; 330) rohrförmig in den Grundkörper (212; 312) eingelassen ist.

2. Hohlprofileinsteckstopfen (10; 110; 210; 310) nach Anspruch 1, **dadurch gekennzeichnet, dass** die Einsenkung (22; 122; 222; 322) umlaufend ausgebildet ist.

3. Hohlprofileinsteckstopfen (10; 110; 210; 310) nach Anspruch 1 oder Anspruch 2, **dadurch gekennzeichnet, dass** der Hohlprofileinsteckstopfen (10; 110; 210; 310) einstückig ausgebildet ist.

4. Hohlprofileinsteckstopfen (10; 110; 210; 310) nach einem der vorhergehenden Ansprüche, **dadurch gekennzeichnet, dass** der Hohlprofileinsteckstopfen (10; 110; 210; 310) als Spritzgussteil ausgebildet ist.

5. Hohlprofileinsteckstopfen (10; 110; 210; 310) nach einem der vorhergehenden Ansprüche, **dadurch gekennzeichnet, dass** der Hohlprofileinsteckstopfen (10; 110; 210; 310) zumindest teilweise aus Kunststoff ausgebildet ist, insbesondere als Kunststoffspritzgussteil ausgebildet ist.

6. Hohlprofileinsteckstopfen (10; 110; 210; 310) nach Anspruch 5, **dadurch gekennzeichnet, dass** der Kunststoff ein elastischer und/oder flexibler Kunststoff ist.

7. Hohlprofileinsteckstopfen (10; 110; 210; 310) nach einem der vorhergehenden Ansprüche, **dadurch gekennzeichnet, dass** auf wenigstens einer Außenfläche (14; 114; 214; 314) des Grundkörpers (12; 112; 212; 312) eine Verrastungsnoppe (16; 116; 216; 316) angeordnet ist.

## Claims

1. A hollow-profile insertion plug (10; 110; 210; 310) with a basic body (12; 112; 212; 312) for inserting into an open end of a hollow profile and with a covering side (18; 118a; 118b; 218; 318a; 318b) for covering the open end of the hollow profile, wherein the covering side (18; 118a; 118b; 218; 318a; 318b) is moulded onto the basic body (12; 112; 212; 312) and has an encircling protrusion (20; 120; 220; 320), wherein a depression (22; 122; 222; 322) is provided adjoining the protrusion (20; 120; 220; 320) in the transition to the adjoining outer surface (14; 114; 214; 314) of the basic body (12; 112; 212; 312),
wherein a rod receptacle (30; 130; 230; 330) is formed in the basic body (12; 112; 212; 312),
and wherein the rod receptacle (230; 330) is set into the basic body (212; 312) in tubular manner.

2. A hollow-profile insertion plug (10; 110; 210; 310) according to Claim 1, **characterised in that** the depression (22; 122; 222; 322) is formed in encircling manner.

3. A hollow-profile insertion plug (10; 110; 210; 310) according to Claim 1 or Claim 2, **characterised in that** the hollow-profile insertion plug (10; 110; 210; 310) is formed in one piece.

4. A hollow-profile insertion plug (10; 110; 210; 310) according to one of the preceding claims, **characterised in that** the hollow-profile insertion plug (10; 110; 210; 310) is formed as an injection-moulded part.

5. A hollow-profile insertion plug (10; 110; 210; 310) according to one of the preceding claims, **characterised in that** the hollow-profile insertion plug (10; 110; 210; 310) is formed at least partially from plastics material, in particular is formed as an injection-moulded plastics material part.

6. A hollow-profile insertion plug (10; 110; 210; 310) according to Claim 5, **characterised in that** the plastics material is an elastic and/or flexible plastics material.

7. A hollow-profile insertion plug (10; 110; 210; 310) according to one of the preceding claims, **characterised in that** a latching knob (16; 116; 216; 316) is arranged on at least one outer surface (14; 114; 214; 314) of the basic body (12; 112; 212; 312).

## Revendications

1. Bouchon d'insertion pour profilé creux (10 ; 110 ; 210 ; 310) doté d'un corps de base (12 ; 112 ; 212 ; 312) à insérer dans une extrémité ouverte d'un profilé creux et d'un côté de recouvrement (18; 118a; 118b; 218; 318a; 318b) destiné à recouvrir l'extrémité ouverte du profilé creux ;
le côté de recouvrement (18 ; 118a ; 118b ; 218 ; 318a ; 318b) étant moulé contre le corps de base (12 ; 112 ; 212 ; 312) et ayant une saillie (20 ; 120 ; 220 ; 320) périphérique, une creusure (22 ; 122 ; 222 ; 322) étant prévue à côté de la saillie (20 ; 120 ; 220 ; 320) dans la zone de transition d'avec la surface extérieure (14 ; 114 ; 214 ; 314) connexe du corps de base (12; 112 ; 212 ; 312) ;
un logement de tige (30 ; 130 ; 230 ; 330) étant réalisé dans le corps de base (12 ; 112 ; 212 ; 312) ; et
le logement de tige (230 ; 330) étant encastré en forme de tube dans le corps de base (212 ; 312).

2. Bouchon d'insertion pour profilé creux (10; 110 ; 210; 310) selon la revendication 1, **caractérisé en ce que** la creusure (22 ; 122 ; 222 ; 322) est réalisée de façon périphérique.

3. Bouchon d'insertion pour profilé creux (10; 110; 210; 310) selon la revendication 1 ou 2, **caractérisé en ce que** le bouchon d'insertion pour profilé creux (10 ; 110 ; 210 ; 310) est réalisé d'un seul tenant.

4. Bouchon d'insertion pour profilé creux (10; 110; 210 ; 310) selon l'une quelconque des revendications précédentes, **caractérisé en ce que** le bouchon d'insertion pour profilé creux (10 ; 110 ; 210 ; 310) est réalisé sous la forme d'une pièce moulée par injection.

5. Bouchon d'insertion pour profilé creux (10; 110; 210 ; 310) selon l'une quelconque des revendications précédentes, **caractérisé en ce que** le bouchon d'insertion pour profilé creux (10; 110; 210 ; 310) est réalisé au moins en partie en matière plastique, notamment sous la forme d'une pièce moulée par injection en matière plastique.

6. Bouchon d'insertion pour profilé creux (10; 110; 210; 310) selon la revendication 5, **caractérisé en ce que** la matière plastique est une matière plastique élastique et/ou flexible.

7. Bouchon d'insertion pour profilé creux (10; 110; 210 ; 310) selon l'une quelconque des revendications précédentes, **caractérisé en ce qu'**un picot d'arrêt (16 ; 116 ; 216 ; 316) est disposé sur au moins une surface extérieure (14 ; 114 ; 214 ; 314) du corps de base (12 ; 112 ; 212 ; 312).
